# DEMANDE DE BREVET EUROPEEN

(11) **EP 1 352 953 A2**
(43) Date de publication de la demande: **15.10.2003**
(21) Numéro de dépôt: 03356056.6
(22) Date de dépôt: 27.03.2003
(51) Int. Cl.: C12N 1/14, C02F 3/34, C02F 11/02, C02F 3/30

(54) **Inoculum fongique, procédés de préparation dudit inoculum et procédés de mise en oeuvre dans le traitement d'effuents riches en matière organique**

(30) Priorité: 27.03.2002 FR 0203843
(71) Demandeur: Biovitis S.A., 15400 Saint-Etienne-De-Chomeil (FR)
(72) Inventeur: Berthon, Jean-Yves, 69170 Perignat Les Sarlieve (FR); Grizard, Damien, 63000 Clermont-Ferrand (FR)
(74) Mandataire: Hinterberg, Katherine

(57) **Abrégé**

Inoculum fongique utilisable pour le traitement d'effluents riches en matières organiques, caractérisé en ce qu'il comporte en combinaison au moins deux souches de champignons filamenteux choisies chacune dans le groupe constitué par les champignons du genre *Mucor* et les champignons du genre *Geotrichum*

Procédé de préparation dudit inoculum fongique et procédés de mise en oeuvre.

## Description

La présente invention a trait au domaine du traitement des effluents riches en matières organiques.

Le problème le plus aigu est notamment le problème du traitement des lisiers issus des élevages industriels, souvent hors sol.

Les espèces animales concernées sont principalement les porcs mais également selon les régions les veaux de boucherie, les vaches laitières, les gallinacés comme les poules pondeuses, ou encore les palmipèdes comme les canards.

Les effluents se présentent pour la plupart du temps sous forme liquide, le lisier, comportant éventuellement une part variable de teneur en matière organique solide.

Le problème de la gestion agronomique rationnelle de ceux-ci se pose de plus en plus souvent. La principale difficulté est de valoriser ces effluents par épandage sur des surfaces agricoles, à des doses et des formes chimiques compatibles avec les exigences agronomiques, tout en prévenant les risques majeurs de pollution de l'eau, de l'air associés à des nuisances liées aux mauvaises odeurs. Actuellement, la législation européenne ne porte que sur la quantité d'azote (donc de nitrates) épandue, limitée à 170 kg par hectare et par an. Cependant, le phosphore est actuellement particulièrement surveillé, car sa forte concentration dans les déjections le rend excédentaire, même lorsque l'apport d'azote est ajusté aux besoins des plantes.

Les filières « classiques » de traitements permettant de prévenir les pollutions restent majoritairement basées sur :
- une concentration du phosphore dans des résidus solides, son exportation et son utilisation comme fertilisant hors des zones de production intensive.
- une élimination de l'azote par nitrification-dénitrification endogènes (traitement biologique apparenté aux méthodes utilisées dans les stations d'épuration des eaux usées).

Les filières biologiques globales de traitement sont généralement caractérisées par les deux étapes suivantes :
- une séparation de phase
- un traitement biologique

La séparation de phases vise principalement à retenir la majorité du phosphore, essentiellement présent dans le lisier sous forme de particules, ainsi qu'une quantité importante de matières organiques et à les exporter ultérieurement dans des coproduits solides d'intérêt agronomiques. L'exportation peut exiger selon les cas, une étape supplémentaire de compostage ou « stabilisation aérobie » du résidu solide. Cette étape débute par un transfert du lisier, des pré-fosses vers une cuve ouverte d'homogénéisation. L'homogénéisation est réalisée par simple agitation des lisiers pendant un à deux jours, à température ambiante, et vise à délivrer un débit constant de lisier homogène.

En fonction des rendements de rétention du phosphore dans la phase solide, escomptés (entre 15 % et 90 %), différentes techniques de séparation peuvent être envisagées. Il s'agit principalement de :
- décantation statique dans une cuve ouverte non-agitée,
- décantation accompagnant un processus de dégradation partielle des constituants carbonées dans une cuve totalement anaérobie. La séparation matières, dans ce cas, n'est pas la finalité. En effet, l'objectif principal est d'acidifier le lisier afin de liquéfier un maximum de matières solides. Néanmoins, cette acétogénèse réalisée dans une enceinte non agitée provoque obligatoirement une décantation des matières solides les plus compactes et riches en minéraux.
- filtration sur végétaux
- tamisage, vis compacteuse
- centrifugation avec ou sans floculation.

L'étape de traitement biologique est caractérisée par une volatilisation de l'azote sous forme d'azote atmosphérique (N₂). Ce traitement exploite les systèmes bactériens nitrificateurs et dénitrificateurs présents à l'état naturel dans les effluents. Au cours d'une première étape se déroulant en conditions aérobies (en présente d'oxygène), des bactéries naturellement présentes dans le lisier, essentiellement des *nitrosomonas* et des *nitrobacter,* oxydent l'azote ammoniacal en nitrates. Puis, dans une deuxième étape, en l'absence d'oxygène, des micro-organismes dénitrifiants (*Pseudomonas, Paracoccus, Bacillus,* ...) réduisent ces nitrates en azote gazeux, élément inoffensif puisqu'il constitue 80% de l'atmosphère. Les deux phases peuvent se dérouler dans deux cuves distinctes ou, plus fréquemment, dans une même enceinte soumise successivement à des phases aérées, par agitation ou insufflation d'air, et non aérées.

Les effluents appauvris en phosphore et en azote, généralement stockés dans des lagunes, peuvent être utilisés en épandage ou en irrigation sur des parcelles cultivées.

Cependant, la phase liquide libérée lors de la séparation de matières possède souvent une concentration en matière organique qui est excédentaire par rapport aux capacités épuratoires naturelles des systèmes bactériens mis en oeuvre dans l'étape de traitement biologique. En fait, seule une faible part de cette charge carbonée et azotée peut être utilisée comme donneur d'électrons par les bactéries dénitrifiantes lors de la réduction des nitrates en azote atmosphérique. La fraction non-métabolisée constitue donc une source importante de composés malodorants (par exemple, formation à partir de molécules organiques complexes d'acides organiques à courte chaîne responsables des odeurs les plus agressives et une source de pollution en cas d'épandage.

On connaît de FR 2 796 376 un procédé de biodigestion par ensemencement par mycélium des effluents à traiter lors de l'étape de traitement biologique.

La mise en oeuvre de ce procédé implique un ensemencement préalable de la cuve de rétention et de la tour qui la surmonte par les mycéliums et la mise en place d'un système de recirculation permanente de l'effluent sur le garnissage de la tour pour permettre la biodigestion des matières organiques de l'effluent par lesdits mycéliums.

Ce procédé implique donc la mise en place d'une installation spécifique.

La présente invention concerne un inoculum fongique utilisable pour le traitement d'effluents riches en matières organiques, caractérisé en ce qu'il comporte en combinaison au moins deux souches de champignons filamenteux.

Cet inoculum fongique constitué d'une biomasse qui peut se présenter sous forme de solution ou fixée sur des supports solides fixes ou particulaires, peut être utilisé quelques soient les installations mises en oeuvre pour le traitement des effluents riches en matières organiques et quels que soient ces effluents, par ensemencement direct des cuves contenant les effluents soumis à l'étape de traitement biologique, la condition essentielle étant que cette étape soit effectuée en condition aérobie.

L'ajout de cet inoculum fongique au démarrage de l'étape de traitement biologique permet ainsi que l'action épuratoire des champignons filamenteux, la nitrification et la dénitrification par la flore bactérienne se déroulent dans la même enceinte, dans les mêmes conditions physico-chimiques et simultanément.

L'ajout de cet inoculum fongique peut également être réalisé au moment du compostage des résidus solides des lisiers.

Les champignons sont des organismes eucaryotes, sans chlorophylle et dont l'appareil végétatif, dépourvu de tiges, de racines et de feuilles est appelé thalle. Le thalle peut être unicellulaire, dissocié et bourgeonnant (levures), ou filamenteux (champignons filamenteux ou moisissures), habituellement limité par une paroi rigide. Le nombre de champignons filamenteux est, à l'heure actuelle, estimé à environ 50 000 espèces connues et au moins autant à découvrir.

Les champignons filamenteux (moisissures) se différencient des procaryotes (bactéries) par le fait que leurs cellules sont généralement de taille supérieure, contenant un noyau, des vacuoles et des mitochondries, ce qui est spécifique aux cellules eucaryotes.

Les champignons sont incapables d'effectuer la photosynthèse et tirent leur énergie de l'oxydation des composés chimiques organiques. Ils sont immobiles à cause de la rigidité de leur paroi et ont une structure mycélienne constituée de filaments ramifiés, les hyphes, qui constituent le mycélium.

Dotés d'un arsenal enzymatique très varié, les champignons filamenteux sont capables de coloniser tous les milieux et sont capables pour certaines souches de tolérer des conditions extrêmes de pH qui peut être compris entre 3,5 et 9 par exemple, de températures de 0°C à plus de 40°C et même des teneurs en eau très faibles.

Les champignons présentent des aptitudes à réaliser certaines fonctions biochimiques de dégradation vis-à-vis des différents cycles biologiques des éléments illustrés dans le tableau suivant :

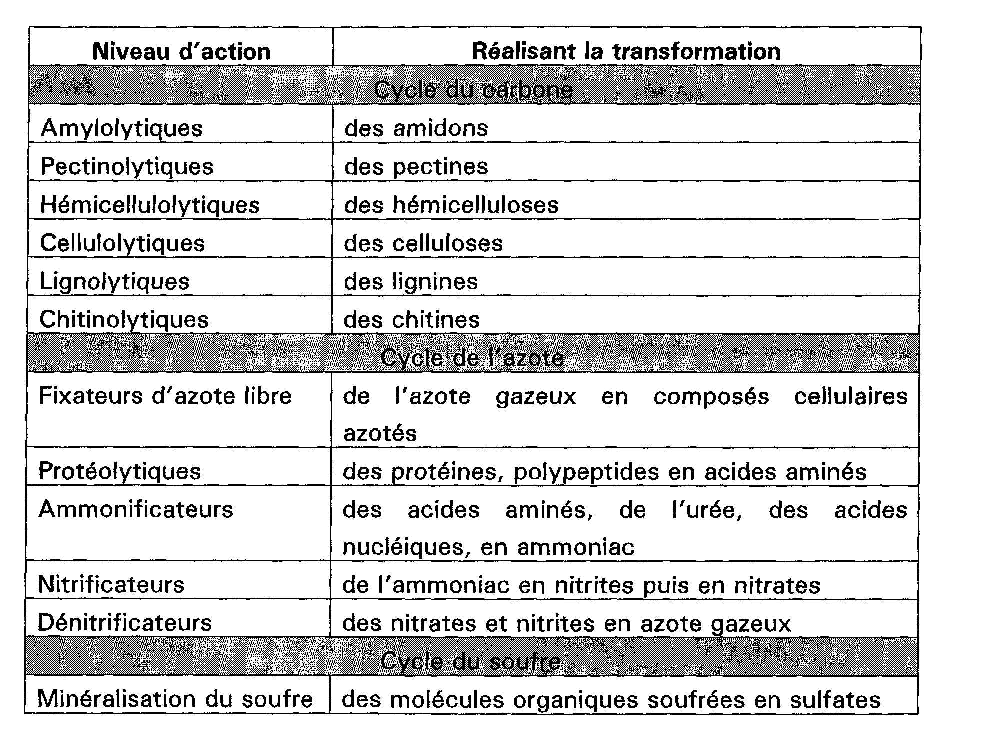

Ces réactions de transformation de la matière organique sont dues à des productions de protéines spéciales par les champignons filamenteux : les enzymes, véritables biocatalyseurs de toutes les réactions biochimiques. Les principales activités enzymatiques identifiées dans les champignons filamenteux figurent dans le tableau suivant :

| **1 - hydrolases** | Dégradation d'une molécule du susbstrat par l'eau et fixation (-H et -OH) |
|---|---|
| Osidases : | Attaque des osides |
| - saccharase | saccharose → glucose + fructose |
| - amylase | amidon → dextrine + maltose |
| - cellulase | cellulose → sucres simples + cellulodextrines |
| - pectinase | pectines → ac.galacturonique + galactose |
| - phosphatases | alcool-phosphate→alcool + phosphate |
| | |
| Amidases : | Destruction amide |
| - uréase | urée → ammoniac |
| - asparaginase | asparagine→ac.aspartique + ammoniac |
| Protéases | protéines →acides aminés ou peptides |

| **2 - Transférases et isomérases** | Transfert des groupes ou des radicaux d'une molécule à une autre ou changement de configuration |
|---|---|
| 3 - **Oxydo-réductases** | Oxydo-réduction |
| | |
| - osydases et catalases | Réduction d'oxygène gazeux |
| - hydrogénases | |

| 4 - **Clastases ou synthétases** | Rupture ou formation d'une liaison |
|---|---|
| | |

Grâce aux divers systèmes enzymatiques précédemment évoqués, les différents éléments constitutifs de base du lisier (C, O, N, H e P) peuvent donc être en grande partie assimilés ou transformés.

L'expression de ces activités enzymatiques via les champignons filamenteux va permettre de modifier la charge organique présente dans les effluents durant tout le traitement biologique.

L'inoculation des effluents par des champignons filamenteux assure la décomposition des molécules organiques en excès. En retour, la bio-destruction de ces composés va éviter la formation de composés malodorants résiduels aussi bien au moment du stockage en lagune qu'au moment de l'épandage ou de l'irrigation. Ainsi, l'utilisation d'un inoculum fongique permet une élimination durable des nuisances olfactives, sans émission d'odeur post-traitement.

La protéolyse (transformation de molécules azotées complexes en ammoniaque) du résidu organique entraîne également une libération permanente d'ammoniaque, directement oxydable en nitrates par les systèmes nitrifiants endogènes. Cet apport constant d'ammoniaque va donc maintenir le traitement biologique à un niveau d'activité optimum. En conséquence, les champignons filamenteux participent à la réduction de la charge azotée et leur présence permet l'obtention de rendements d'élimination d'azote gazeux supérieurs à 70 %.

L'expression des activités enzymatiques précédemment décrites via les champignons filamenteux, permet également une activation des processus de compostage des résidus solides obtenus lors des séparations matière en début de traitement.

La présente invention concerne ainsi un inoculum fongique utilisable pour le traitement d'effluents riches en matières organiques, caractérisé en ce qu'il comporte en combinaison au moins deux souches de champignons filamenteux choisies chacune dans le groupe constitué par les champignons du genre *Mucor* et les champignons du genre *Geotrichum.*

Plus particulièrement, l'invention concerne un inoculum fongique caractérisé en ce la souche de champignons filamenteux appartenant au genre *Mucor* est une souche de *Mucor hiemalis* telle que déposée sous le n° CNCM 1-2824 auprès de la CNCM le 22 mars 2002, et/ou une souche de *Mucor racemosus.*

Plus particulièrement, l'invention concerne un inoculum fongique caractérisé en ce la souche de champignons filamenteux appartenant au genre *Geotrichum* est une souche de *Galactomyces* *geotricum* telle que déposée sous le n° CNCM 1-2825 auprès de la CNCM le 22 mars 2002, ou une souche de *Geotrichum klebahnii*.

L'inoculum fongique peut comprendre en outre une ou plusieurs souches de champignons filamenteux choisis dans le groupe constitué par les champignons appartenant aux genres *Aspergillus, Fusarium, Trichoderma, Phoma, Rhizopus et*/*ou Scedosporium*

Dans un mode de réailisation, la souche de champignons appartenant au genre *Fusarium* est une souche de *Fusarium graminearum* ou une souche de *Fusarium equiseti* telle que déposée sous le n° CNCM I-2821 le 22 mars 2002 auprès de la CNCM, la souche de champignons appartenant au genre *Trichoderma* est une souche de *Trichoderma viride* telle que déposée sous le n° CNCM I-2822 auprès de la CNCM le 22 mars 2002, ou une souche de *Trichoderma harzianum,* la souche de champignons appartenant au genre *Phoma* est une souche de *Phoma sp.* telle que déposée sous le n° CNCM I-2823 auprès de la CNCM le 22 mars 2002, la souche de champignons appartenant au genre *Aspergillus* est une souche *d'Aspergillus phoenicis* telle que déposée sous le n° CNCM 1-2826 auprès de la CNCM le 22 mars 2002, la souche de champignons appartenant au genre *Rhizopus* est une souche de *Rhizopus stolonifer* et la souche de champignons appartenant au genre *Scedosporium* est une souche de *Scedosporium agrospermum.*

L'inoculum fongique peut en outre comprendre des champignons filamenteux comme le *Chaetomium globosum* ou des *Penicillium* comme le *Pénicillium granulatum*, le *Pénicillium verrucosum* ou le *Pénicillium chrysogenum* ou des *Paecilomyces* comme le *Paecilomyces variotii*.

Les souches de champignons filamenteux citées précedemment peuvent être obtenues par commande par exemple en Europe auprès des organismes suivants :
BCCM/MUCL AGRO-INDUSTRIAL FUNGI AND YEASTS COLLECTION, Université Catholique de LOUVAIN-LA-NEUVE, Place CROIX de SUD 3, B1378 LOUVAIN-LA -NEUVE, BE, et
CBS, Centraal Bureau voor Schimmelcultures, Oosterstraat 1, P.O. Box 273, 3740 AG BAARN, NL.

La caractérisation des champignons lorsque ceux-ci sont cultivés à partir de souches indigènes peut être effectuée soit par consultation des mycothèques par exemple la mycothèque de la BCCM/MUCL ci-dessus citée soit par référence aux ouvrages suivants :
The Mushroom Cultivator, P. Stamets, J.S. Chilton, AGARIKON PRESS, Olympia, Washington,
Smith's Introduction to Industrial Mycology, 7^{th} Ed., A.H.S. Onions, D. Allsopp, H.O.W. Eggins, EDWARD ARNOLD Ltd, LONDON, et
Moississures utiles et nuisisbles, Importance Industrielle, B. BOTTON et al., MASSON, PARIS, 1990.

Les caractéristiques essentielles des genres auxquels appartiennent les souches de champignons citées dans la présente demande sont reprises ci-après.

Par champignon du groupe *Aspergillus*, on entend tout champignon ayant les caractéristiques suivantes :
Thalle a mycélium cloisonné portant de nombreux conidiophores dressés, non ramifiés, terminés en en vésicules. Phialides formées directement sur la vésicule (têtes conidiennes unisériées) ou portées sur des métules ou stérigmates (têtes conidiennes bisériées). Conidies sèches, en chaînes divergentes ou associées en colonnes compactes, unicellulaires, globuleuses, sub-globuleuses ou elliptiques, lisses ou ornementées, hyalines ou pigmentées en jaune, brun, noir ou vert. Cellules à paroi épaissie (Hülle cells) et sclérotes parfois présents.

Par champignon du groupe *Fusarium*, on entend tout champignon ayant les caractéristiques suivantes :
Thalle à croissance généralement rapide, blanc à crème, jaune, brunâtre, rose, rouge, violet ou lilas. Conidiophores parfois très ramifiés formant sur le thalle des coussinets (sporodochies) et portant des masses de spores d'aspect graisseux. Phialides plus ou moins allongées pouvant produire deux types de conidies : des macroconidies fusiformes, souvent courbées, pluriseptées, avec une cellule basale pédicellée, portant une sorte de talon ; des microconidies petites, O-2-septées, piriformes, fusiformes ou ovoïdes (certaines espèces produisent les deux types de spores, d'autres ne forment que des macroconidies). Chlamydospores présentes ou absentes, terminales ou intercalaires, différenciées par le mycélium ou par les conidies.

Par champignon du groupe *Geotrichum*, on entend tout champignon ayant les caractéristiques suivantes :
Thalle à croissance rapide, mince, lisse, blanc, présentant un aspect de levure. Arthrospores cylindriques, arrondies aux extrémités, de taille variable, 6-25 x 3-9 µm.

Par champignon du groupe *Mucor*, on entend tout champignon ayant les caractéristiques suivantes :
Thalle siphoné, velouté ou floconneux, blanc, gris ou noir. Pas de stolons. Sporocystophores dressés, toujours terminés par un sporocyste, simples ou à ramification sympodiale, monopodiale ou mixte. Sporocytes globuleux, blancs ou un peu colorés, sans apophyse, pourvus d'une columelle, multisporés. Spores de forme variée, lisses ou granuleuses. Chlamydospores parfois présentes. Zygospores sans appendices sur les suspenseurs.

Par champignon du groupe *Phoma*, on entend tout champignon ayant les caractéristiques suivantes :
Sphaeropsidale à pycnides noires. Conidies unicellulaires, ovoïdes à elliptiques, produites sur des phialides, exsudées par l'ostiole où elles s'agglutinent en une mèche parfois très longue.

Par champignon du groupe *Rhizopus*, on entend tout champignon ayant les caractéristiques suivantes :
Thalle à croissance extrêmement rapide, très luxuriant, capable de se développer, grâce à des stolons, sur les parois des récipients de culture ou entre le couvercle et le fond des boîtes de Petri, contaminant ainsi l'extérieur par ses spores. Sporocystophores pigmentés, généralement très grands, terminés en entonnoir (apophyse), isolés ou en bouquet de 2-6 présentant à la base des rhizoïdes noirs. Columelles brunes, globuleuses ou semi-globuleuses. Spores ellipsoïdales, lisses ou striées, souvent cabossées.

Par champignon du groupe *Trichoderma*, on entend tout champignon ayant les caractéristiques suivantes :
Thalle à croissance rapide, d'abord lisse puis plus ou moins floconneux, souvent zoné, blanc ou vert. Conidiophores en touffes plus ou moins compactes, très ramifiés, irrégulièrement verticillés avec des ramifications à angle droit. Phialides ovoïdes à ellipsoïdales, atténuées au sommet, solitaires ou en groupe, généralement perpendiculaires à l'axe. Conidies réunies en glomérules au sommet des phialides, unicellulaires, parfois hyalines, le plus souvent vertes, lisses ou granuleuses. Clamydospores parfois présentes.

Par champignon du groupe *Scedosporium*, on entend des hyphomycètes aleuriosporés assez répandus dans la nature, principalement telluriques. Leur croissance sur malt gélosé à 25-30°C pendant 5 à 6 jours montre un mycélium laineux, blanchâtre en début de culture, puis gris-brun à revers très sombre. Les éléments de la multiplication asexuée se composent d'hyphes septées, fines et enchevêtrées, portant directement des conidies ou au contraire des conidiophores, quelquefois groupés en corémies, générant des aleuriospores brunes et ovoïdes à base tronquée.

L'invention concerne également les souches de champignons filamenteux suivantes :
souche de l'espèce *mucor hiemalis* telle que déposée sous le n° CNCM 1-2824 auprès de la CNCM le 22 mars 2002, souche de l'espèce *Galactomyces geotrichum* telle que déposée sous le n° CNCM 1-2825 auprès de la CNCM le 22 mars 2002, souche de l'espèce *Aspergillus phoenicis* telle que déposée sous le n° CNCM 1-2826 auprès de la CNCM le 22 mars 2002, souche de l'espèce *Fusarium equiseti* telle que déposée sous le n° CNCM 1-2821 auprès de la CNCM le 22 mars 2002, souche de l'espèce *Trichoderma viride* telle que déposée sous le n° CNCM 1-2822 auprès de la CNCM le 22 mars 2002, souche de l'espèce *Phoma sp.* telle que déposée sous le n° CNCM 1-2823 auprès dela CNCM le 22 mars 2002.

Elle concerne également tout champignon filamenteux suceptible d'être obtenu par culture de l'une quelconque des souches précédemmetn identifiées.

L'invention concerne également un kit d'inoculation pour le traitement d'effluents riches en matières organiques, caractérisé en ce qu'il est constitué d'un mélange d'au moins deux souches de champignons filamenteux.

Dans une variante de l'invention, ledit kit d'inoculation est constitué d'une solution contenant un mélange des deux souches de champignons filamenteux en quantités égales.

Dans un autre mode de réalisation, lesdites souches de champignons filamenteux sont fixées sur un substrat solide poreux à l'état unitaire ou divisionnaire, permettant de favoriser leur colonisation après inoculation du milieu à traiter.

L'invention concerne également un procédé de préparation d'un inoculum fongique comprenant une combinaison d'au moins deux souches de champignons filamenteux, caractérisé en ce qu'il comporte les étapes suivantes :
- mise en suspension d'un échantillon de milieu susceptible de contenir les espèces nécessaires à la préparation de l'inoculum fongique,
- homogénéisation de l'échantillon,
- dilution de l'échantillon,
- culture, isolement et caractérisation des colonies,
- récupération de la biomasse,
- dilution et conditionnement,
- choix des souches isolées,
- combinaison des souches choisies,
- conditionnement de l'inoculum primaire préparé.

Dans un mode de réalisation préféré, l'inoculum primaire est conservé par congélation de la biomasse après dilution dans un tube cryogénique.

Par exemple, les inoculums peuvent être obtenus en réalisant des cultures avec des milieux de routine, peu sélectifs tels que le milieu de Czapek, le milieu glucosé à l'extrait de levure ou milieu de Mossel, le milieu P.D.A. (Potato Dextrose Agar), des milieux maltés ou le milieu de Sabouraud. Les températures d'incubation peuvent être comprises entre 20°C et 30°C.

L'invention concerne également la préparation d'un inoculum industriel contenant de 10⁴ à 10⁷ Thalles ou unités viables par ml de solution à partir des inoculums primaires, c'est-à-dire de la biomasse récoltée et préalablement conditionnée. Cet inoculum industriel est préparé par dilutions successives et mises en culture successives à partir de l'inoculum primaire.

Ce procédé de préparation d'inoculums industriels permet d'éviter la dégénérescence des souches utilisées.

L'invention concerne également l'utilisation d'un mélange de champignons filamenteux choisis parmi les souches indigènes présentes dans un effluent riche en matières organiques, ayant éventuellement subi une première phase de traitement biologique par une flore anaérobie, pour la dégradation en phase aérobie des composés azotés et de la pollution organique présente et éventuellement résiduelle dans ledit effluent.

L'invention concerne également le procédé de traitement d'effluents riches en matières organiques par ensemencement par un inoculum fongique.

Ce procédé de traitement peut être intégré directement dans les filières d'épuration existantes basées sur l'élimination de l'azote par nitrification et dénitrification et sur la gestion des flux de phosphore par séparation de phases.

Il permet en outre de disposer après traitement d'une eau d'irrigation dont la charge minérale résiduelle est assimilable durant toute la période de croissance des plantes, d'activer le compostage du résidu solide obtenu lors des séparations matières.

Il permet également de potentialiser les systèmes de traitement déjà en place en améliorant l'élimination de l'azote avec un rendement de dégradation azotée supérieur à 70 %.

Il permet de dégrader la matière organique en excès qui ne peut pas être utilisée par les systèmes de dénitrification par les bactéries présentes dans le milieu à traiter.

Le rendement de dégradation de la Demande Biochimique en Oxygène (DBO) est de 90 %.

Il permet ainsi d'éliminer les nuisances olfactives liées aux effluents liquides de lisiers, relarguées par les filières aussi bien au niveau des élevages qu'au moment des épandages.

Dans une variante de réalisation, le procédé selon l'invention est un procédé de traitement d'effluents riches en matières organiques comportant au moins une phase de traitement biologique aérobie, caractérisé en ce que le traitement biologique est effectué par ensemencement par un inoculum fongique comportant au moins deux souches de champignons filamenteux.

Les champignons filamenteux sont choisis parmi les champignons filamenteux appartenant au groupe des *Mucor* et des G*eotrichum*.

Dans une variante de mise en oeuvre, les souches de champignons filamenteux appartiennent aux espèces fongiques indigènes du milieu à traiter.

L'invention concerne également un procédé de traitement d'effluents riches en matières organiques caractérisé en ce qu'il comporte les étapes suivantes :
- prélèvement d'un échantillon du milieu à traiter,
- isolement des souches de champignons filamenteux présents dans le milieu à traiter,
- culture des souches isolées,
- récupération des biomasses correspondant aux souches isolées,
- préparation d'un inoculum à partir des biomasses récupérées,
- ensemencement des effluents à traiter par l'inoculum préparé.

Lorsque le procédé est intégré à une filière classique de nitrification-dénitrification, il est caractérisé en ce qu'il comporte deux phases de traitement biologique, une première phase de traitement par une flore bactérienne anaérobie et une seconde phase de traitement par les champignons filamenteux. Dans cette variante, le procédé comprend les étapes suivantes :
a) transfert des effluents dans une installation de décantation et de traitement des matières décantées par une flore anaérobie,
b) transfert de l'effluent obtenu à l'étape a) dans une seconde installation de rétention et de traitement dudit effluent par des champignons filamenteux en présence d'oxygène,
c) évacuation de l'effluent obtenu à l'étape b).

Dans cette variante de procédé, les champignons filamenteux sont choisis dans le groupe constitué par les *Mucor*, les G*eotrichum,* les A*spergillus*, les F*usarium,* les T*richoderma,* les P*homa,* les R*hizopus,* les S*cedosporium.*

Les procédés de préparation et de mise en oeuvre des inoculums fongiques sont illustrés par les exemples suivants :

### 1) Isolement de souches indigènes et mise en collection.

Les isolements sont réalisés en trois étapes :
1^{ère} étape :
   4 g (ou 4 ml) d'échantillon du milieu à traiter sont mis en solution ou en suspension dans 36 g de solution diluante (dilution 10⁻¹).
   La solution diluante est de préférence une solution aqueuse, de 2 g de peptone de caséine, de 9 g de chlorure de sodium contenant 5 ml de TWEEN dans un litre d'eau déminéralisée qui est stérilisée préalablement à son utilisation par autoclavage pendant 15 à 20 minutes à 118-122°C.
   La solution et/ou la suspension obtenue est homogénéisée par agitation.
   Une série de dilutions successives sont effectuées de 10⁻¹ à 10⁻ ⁶ immédiatement après la mise en solution ou en suspension.
   Des milieux de culture contenus dans des boîtes de diamètre 145 mm sont inoculés avec 0,1 ml de solution obtenue.
   Les milieux de culture utilisés sont :
   - 2 boîtes de PDA (Potato Dextrose Agar, Biokar Diagnostics, BK 095)
   - 2 boîtes d'YGC (Chloramphenicol Glucose Agar, Biokar Diagnostics, BK 007HA)
   - 2 boîtes de Sabouraud + Chloramphénicol (Sabouraud Chloramphenicol Agar, Biokar Diagnostics, BK 027HA).

   L'incubation est maintenue pendant 4 jours à 25°C.
2^{ème} étape :
   Les colonies obtenues à l'étape 1 sont sélectionnées visuellement et prélevées.
   Cette sélection est effectuée après caractérisaition des souches soit par consultation d'un mycothèque telle que précédemment citée soit par consultation des ouvrages de référence précédemment cités.
   Après prélèvement, ces colonies sont diluées dans la solution diluante (de 10⁻¹ à 10⁻⁶). Des milieux de culture identiques à ceux utilisés dans l'étape 1 sont inoculés avec 0,1 ml de solution obtenue précédemment.
   L'incubation est maintenue pendant 4 jours à 25°C.
3^{ème} étape :
   A partir de prélèvements dilués comme dans l'étape 2, des milieux de culture Sabouraud + Chloramphénicol sont inoculés par piqure centrale et l'incubation est poursuivie.
   On obtient des souches isolées qui sont ensuite mises en culture sur gélose inclinée (Slants sur Sabouraud) pendant 5 jours à 25°C.
   La biomasse est "récoltée" à partir des géloses inclinées et diluée (9 ml de solution diluante par Slants). Le mélange est passé au VORTEX et le surnageant obtenu peut être conditionné par addition à 1,4 ml de surnageant de 0,4 ml de glycérol stérile et congelé à - 80°C, dans des tubes cryogéniques permettant un stockage aisé des souches.

### 2) Préparation d'un inoculum industriel

Les inoculums industriels permettant de traiter de grands volumes d'effluents sont préparés à partir du contenu des tubes cryogéniques précédemment conditionné, par dilution de ce contenu dans 1 à 5 I de milieu liquide de production. Ces milieux liquides de production sont des milieux classiques tels que milieux de CZAPEK ou de MOSSEL ou milieu malté.

Au bout de 4 à 7 jours, il y a un développement des souches et cette production peut ensuite être utilisée comme inoculum secondaire pour la production de centaines de litres d'inoculum industriel par dilution puis mise en culture dans des milieux liquides.

L'inoculum industriel finalement obtenu contient de 10⁴ à 10⁷ Thalles ou unités viables par ml selon les souches utilisées.

Selon les modes de réalistion précédememnt décrits, un inoculum fongique comprenant une souche appartenant à l'espèce *Mucor hiemalis* et une souche appartenant à l'espèce *Galactomyces geotrichum* a été préparé.

4 g de lisier de porcs ont été mis en solution dans 36 g de solution diluante.

Après homogénéisation et mise en culture 4 colonies ont été isolées.

La caractérisaition a été effectuée par consultation de la Mycothèque de la BCCM/MUCL (demande d'identification).

Parmi les 4 échantillons soumis les espèces suivantes ont été identifiées :
*Phoma sp., Galactomyces geotrichum, Mucor hiemalis et Trichoderma harzianum*.

Les colonies correspondant aux espèces *Galactomyces geotrichum* ont été mises en culture selon le mode opératoire de la deuxième étape puis sur Slants selon la deuxième étape.

La biomasse a été récoltée, puis conditionnée dans des tubes cryogéniques.

Le contenu de deux tubes cryogéniques précédemment obtenu, un par espèce, a été ensuite mis en culture successivement dans des mileux liquides de CZAPEK.

On obtient après 7 jours de culture 5 I d'inoculum contenant respectivement 10⁵ (*Mucor hiemalis*) et 10⁷ (*Galactomyces geotrichum)* unités viables par ml.

Ces 5 I d'inoculum seront ensuite utilisé pour préparer plusieurs centaines de litre d'inoculum industriel.

### 3) Inoculation d'un milieu à traiter

Dans une installation de traitement classique comportant une cuve de traitement en phase anaérobie et une cuve de traitement en phase aérobie, les opérations suivantes sont effectuées :

La cuve de traitement en phase anaérobie est alimentée par le milieu à traiter (lisier de porc).

Le milieu séjourne pendant 10 jours dans cette cuve.

L'effluent issu de cette première cuve de traitement est ensuite acheminé vers une deuxième cuve où est ajouté l'inoculum industriel contenant de 10⁴ à 10⁷ Thalles ou unités viables par ml de solution.

L'inoculum est ajouté à raison de 10⁻⁴ à 10⁻² g d'inoculum industriel par g d'effluent à traiter.

L'effluent séjourne pendant 10 jours dans cette cuve.

Il y a colonisation du milieu par les champignons filamenteux.

On obtient un effluent qui est soutiré de la seconde cuve de traitement.

Apès un temps de séjour de 10 jours en phase aérobie, on obtient les résultats suivants après inoculation avec un inoclum préparé à partir de l'inoculum comportant une souche appartenant à l'espèce *Mucor hiemalis* et une souche appartenant à l'espèce *Galactomyces geotrichum*, ci-dessus exemplifié :

| **Paramètres** | **Rendement du traitement** |
|---|---|
| Demande Chimique en Oxygène (DCO) | 90 à 95 % |
| Phosphore total | 90 % |
| Azote total | > 70 % |

## Revendications

1. Inoculum fongique utilisable pour le traitement d'effluents riches en matières organiques, **caractérisé en ce qu'**il comporte en combinaison au moins deux souches de champignons filamenteux choisies chacune dans le groupe constitué par les champignons du genre *Mucor* et les champignons du genre *Geotrichum*

2. Inoculum fongique selon la revendication 1, **caractérisé en ce qu'**il comprend en outre une ou plusieurs souches de champignons filamenteux choisis dans le groupe constitué par les champignons appartenant aux genres *Aspergillus, Fusarium, Trichoderma, Phoma, Rhizopus et*/*ou Scedosporium.*

3. Inoculum selon l'une quelconque des revendications précédentes **caractérisé en ce** la souche de champignons filamenteux appartenant au genre *Mucor* est une souche de *Mucor hiemalis* telle que déposée sous le n° CNCM 1-2824 auprès de la CNCM, et/ou une souche de *Mucor racemosus.*

4. Inoculum selon l'une quelconque des revendications précédentes **caractérisé en ce** la souche de champignons filamenteux appartenant au genre *Geotrichum* est une souche de *Galactomyces geotricum* telle que déposée sous le n° CNCM 1-2825 auprès de la CNCM, ou une souche de *Geotrichum klebahnii*.

5. Inoculum selon l'une quelconque des revendications précédentes **caractérisé en ce que** la souche de champignons appartenant au genre *Fusarium* est une souche de *Fusarium graminearum* ou une souche de *Fusarium equiseti* telle que déposée sous le n° CNCM 1-2821 auprès de la CNCM.

6. Inoculum selon l'une quelconque des revendications précédentes **caractérisé en ce que** la souche de champignons appartenant au genre *Trichoderma* est une souche de *Trichoderma viride* telle que déposée sous le n° CNCM 1-2822 auprès de la CNCM, ou une souche de *Trichoderma harzianum.*

7. Inoculum selon l'une quelconque des revendications précédentes **caractérisé en ce que** la souche de champignons appartenant au genre *Phoma* est une souche de *Phoma sp*. telle que déposée sous le n° CNCM 1-2823 auprès de la CNCM.

8. Inoculum selon l'une quelconque des revendications précédentes **caractérisé en ce que** la souche de champignons appartenant au genre *Rhizopus* est une souche de *Rhizopus stolonifer*.

9. Inoculum selon l'une quelconque des revendications précédentes **caractérisé en ce que** la souche de champignons appartenant au genre *Scedosporium* est lune souche de *Scedosporium agrospermum.*

10. Inoculum selon l'une quelconque des revendications précédentes **caractérisé en ce que** la souche de champignons appartenant au genre *Aspergillus* est une souche d'*Aspergillus phoenicis* telle que déposée sous le n° CNCM 1-2826 auprès de la CNCM.

11. Inoculum fongique selon l'une quelconque des revendications précédentes **caractérisé en ce que** les souches de champignons filamenteux appartiennent aux espèces fongiques indigènes des effluents à traiter.

12. Procédé de préparation d'un inoculum fongique comprenant une combinaison d'au moins deux souches de champignons filamenteux **caractérisé en ce qu'**il comporte les étapes suivantes :
- mise en suspension d'un échantillon de milieu susceptible d contenir les espèces nécessaires à la préparation de l'inoculum fongique,
- homogénéisation de l'échantillon,
- dilution de l'échantillon,
- culture, isolement et caractérisation des colonies
- récupération de la biomasse,
- dilution et conditionnement
- choix des souches isolées
- combinaison des souches choisies,
- conditionnement de l'inoculum primaire préparé.

13. Kit d'inoculation pour le traitement d'effluents riches en matières organiques **caractérisé en ce qu'**il est constitué d'un mélange d'au moins deux souches de champignons filamenteux.

14. Kit selon la revendication 13, **caractérisé en ce que** les souches de champignons filamenteux sont fixées sur un support solide.

15. Procédé de traitement d'effluents riches en matières organiques comportant au moins une phase de traitement biologique aérobie **caractérisée en ce que** le traitement biologique est effectué par ensemencement par un inoculum fongique comportant au moins deux souches de champignons filamenteux.

16. Procédé selon la revendication 15 **caractérisé en ce que** les champignons filamenteux sont choisis parmi les champignons filamenteux appartenant au groupe des *Mucor* et des *Geotrichum.*

17. Procédé selon la revendication 15, **caractérisé en ce que** les souches de champignons filamenteux appartiennent aux espèces fongiques indigènes du milieu à traiter.

18. Utilisation d'un mélange d'au moins deux champignons filamenteux choisis parmi les souches indigènes présentes dans un effluent riche en matières organiques, ayant éventuellement subit une première phase de traitement biologique par une flore anaérobie, pour la dégradation en phase aérobie des composés azotés et de la pollution organique présente et éventuellement résiduelle dans ledit effluent.

19. Procédé de traitement biologique d'effluents riches en matières organiques **caractérisé en ce qu'**il comporte deux phases de traitement biologiques, une première phase de traitement par une flore anaérobie et une seconde phase de traitement par des champignons filamenteux.

20. Procédé selon la revendication 19, **caractérisé en ce qu'**il comprend les étapes suivantes :
a) transfert des effluents dans une installation de décantation et de traitement des matières décantées par une flore anaérobie,
b) transfert de l'effluent obtenu à l'étape a) dans une seconde installation de rétention et de traitement dudit effluent par des champignons filamenteux en présence d'oxygène,
c) évacuation de l'effluent obtenu à l'étape b).

21. Procédé selon l'une quelconque des revendications 19 ou 20, **caractérisé en ce que** les champignons filamenteux sont choisis dans le groupe constitué par les *Mucor,* les *Geotrichum*, les *Aspergillus*, les *Fusarium*, les *Trichoderma*, les *Phoma,* les *Rhizopus*, les *Scedosporium*.

22. Procédé de traitement d'effluents riches en matières organiques, **caractérisé en ce qu'**il comporte les étapes suivantes :
- prélèvement d'un échantillon du milieu à traiter,
- isolement des souches de champignons filamenteux présents dans le milieu à traiter,
- culture des souches isolées,
- récupération des biomasses correspondant aux souches isolées,
- préparation d'un inoculum industriel à partir des biomasses récupérées,
- ensemencement des effluents à traiter par l'inoculum préparé.

23. Souche de champignon filamenteux de l'espèce *mucor hiemalis* telle que déposée sous le n° CNCM 1-2824 auprès de la CNCM.

24. Souche de champignon filamenteux de l'espèce *Galactomyces geotrichum* telle que déposée sous le n° CNCM 1-2825 auprès de la CNCM.

25. Souche de champignon filamenteux de l'espèce *Aspergillus phoenicis* telle que déposée sous le n° CNCM 1-2826 auprès de la CNCM.

26. Souche de champignon filamenteux de l'espèce *Fusarium equiseti* telle que déposée sous le n° CNCM I-2821 auprès de la CNCM.

27. Souche de champignon filamenteux de l'espèce *Trichoderma viride* telle que déposée sous le n° CNCM 1-2822 auprès de la CNCM.

28. Souche de champignon filamenteux de l'espèce *Phoma sp.* telle que déposée sous le n° CNCM 1-2823 auprès dela CNCM.

29. Champignon filamenteux suceptible d'être obtenu par culture de l'une quelconque des souches identifiées aux revendications 23 à 28.

30. Inoculum fongique industriel selon l'une quelconque des revendications 1 ou 2, obtenu à partir d'un inoculum primaire par dilutions et mises en culture successives dans un milieu liquide.

31. Inoculum fongique industriel comportant en combinaison au moins deux souches de champignons filamenteux, **caractérisé en ce qu'**il contient de 10⁻⁴ à 10⁻⁷ Thalles ou unités viables par ml de solution.
